Europäisches Patentamt

European Patent Office

Office européen des brevets

(19)

(11) Publication number: **0 510 483 A1**

# EUROPEAN PATENT APPLICATION

(12)

(21) Application number: **92106374.9**

(51) Int. Cl.⁵: **G01N 33/569**

(22) Date of filing: **14.04.92**

(30) Priority: **22.04.91 US 689407**

(43) Date of publication of application:
**28.10.92 Bulletin  92/44**

(84) Designated Contracting States:
**AT BE CH DE DK ES FR GB GR IT LI LU NL PT SE**

(71) Applicant: **BOEHRINGER INGELHEIM PHARMACEUTICALS INC.**
**900 Ridgebury Road, P.O. Box 368**
**Ridgefield, Connecticut 06877-0368(US)**

(72) Inventor: **Last-Barney, Kathleen**
**333 Union Avenue**
**Mamaroneck, NY 10532(US)**
Inventor: **Merluzzi, Vincent J.**
**RR1 - Box 395**
**Holmes, NY 12531(US)**
Inventor: **Faanes, Ronald**
**P.O. Box 326**
**Pound Ridge, NY 10576(US)**
Inventor: **Marlin, Steven D.**
**18 Terre Haute Road**
**Danbury, CT 06810(US)**

(74) Representative: **Laudien, Dieter, Dr. et al**
**Boehringer Ingelheim GmbH, Abteilung Patente**
**W-6507 Ingelheim am Rhein(DE)**

(54) Method for the detection of viruses.

(57) A method for the detection of a virus or a related group of viruses, in a fluid sample, using a sandwich assay comprising an immobilized primary cell surface receptor for the virus or related group of viruses, and a soluble labelled primary cell surface receptor for the virus or related group of viruses.

FIGURE 1

Biotinylated sICAM−1 (ug/ml)

EP 0 510 483 A1

Field of the Invention

This invention relates to a method for the detection of a virus or a related group of viruses, in a fluid sample, using one or more primary cell surface receptors for the virus. More particularly, this invention relates to a sandwich assay for detecting the presence of a virus or a related group of viruses, in a fluid sample, using an immobilized primary cell surface receptor for the virus or the group of related viruses, and a soluble labelled primary cell surface receptor for the virus or the related viruses.

Background of the Invention

Viruses are important etiological agents of a wide variety of diseases. However, unlike antibiotic treatment for bacterial infections, no general rational therapy has yet been developed for viral infections. In general, vaccines and therapeutic treatments for one virus do not have broad applicability against other viruses or sometimes even against strains of the same virus. Accordingly, determining if a patient is infected with a particular virus or group of viruses, is desirable for designing prophylactic and therapeutic treatment.

For viral infection, a virus must initially attach itself to the surface of the target cell. Receptors on the cell surface facilitate this attachment. Two types of receptors have been reported in the art. One type of receptor, referred to as a primary receptor, mediates the attachment of the virus to the host cell. A primary receptor is present on the specific cell type which is prone to infection with a particular species of virus and, in general, binds only to that particular species of virus. See, e.g., T. Lentz, J. of Gen. Virol. 71: 751 (1990) and T. Lentz, TIPS 9: 247 (July 1988) . The second reported receptor for viruses, referred to as secondary receptors, are not specific to any particular species of virus. The secondary receptor is present on many cells and is involved in the penetration of the virus into the host cell after attachment to the primary receptor. See, e.g., U.S. Patent No. 4,859,769.

Primary receptors for several viruses have been reported in the art. For example, Fisher et al, Nature 331: 76 (1988), report that a primary receptor for HIV is CD4. Weiss et al, Nature 333: 426 (1988), report that a primary receptor for influenza virus haemagglutinin is sialic acid. Mendelsohn et al, Cell 56: 855 (1989), discuss the primary receptor for poliovirus.

Recently it has been shown that soluble intercellular adhesion molecule-I (sICAM-1, a soluble form of the primary receptor for the major subgroup of rhinoviruses, is capable of inhibiting binding and subsequent infection of target cells by major subgroup rhinoviruses. See Marlin et al, Nature 344: 70 (1990) (hereinafter referred to as "Marlin et al"), herein incorporated by reference. Since this major subgroup of rhinoviruses is responsible for about 45-50% of common cold infections, the use of sICAM-1 or derivatives thereof may be beneficial for prophylaxis and treatment. It would be beneficial to have a method of detecting major group rhinoviruses.

Assays for the detection of viruses and diagnosis of viral infections using antibodies and cDNA probes are known. See, e.g., Al-Nakib et al, J. of Med. Virol. 20: 289 (1986) and Al-Nakib et al, J. of Med. Virol. 29: 268 (1989). These assays, however, frequently have drawbacks either in specificity or in usage and are often not suitable for routine diagnosis.

U.S. Patents Nos. 4,376,110 and 4,486,530 describe "two-site" or "sandwich" immunometric assays for the determination of the presence of antigenic substances in fluids using monoclonal antibodies.

It is the purpose of this invention to provide a rapid and reproducible method for the detection of the presence of viruses in fluids, such as the detection of the presence of the major subgroup of rhinoviruses, using primary receptors in a "sandwich" configuration.

Summary of the Invention

This invention relates to a process for the determination of the presence of a virus or a related group of viruses, in a fluid sample, which comprises the steps of:

(a) incubating the fluid sample with an immobilized primary receptor for the virus or the related group of viruses, the immobilized primary receptor being insoluble in the fluid sample, to form a first insoluble complex of the virus or one or more of the related group of viruses, and the immobilized primary receptor;

(b) incubating the first insoluble complex with a labelled soluble primary receptor for the virus or the related group of viruses, to form a final insoluble complex of the labelled soluble receptor, the virus or one or more of the related group of viruses, and the immobilized primary receptor;

(c) separating the final insoluble complex from the fluid sample and any unreacted labelled soluble primary receptor;

(d) measuring either the amount of label associated with the immobilized primary receptor or the amount of unreacted label; and

(e) determining the presence of the virus or related group of viruses, in the fluid sample, by relating the measurement of (d) to a measurement of a reference sample free of the virus or the related group of viruses.

This invention additionally relates to a process for the determination of the presence of a virus or a related group of viruses, in a fluid sample, which comprises the steps of:

(a) incubating the fluid sample with a labelled soluble primary receptor for the virus or the related group of viruses, to form a soluble complex of the virus or one or more of the related group of viruses, and the labelled soluble receptor;

(b) incubating the soluble complex of (a) with an immobilized primary receptor for the virus or the related group of viruses, the immobilized primary receptor being insoluble in the fluid sample, to form a final insoluble complex of the labelled soluble receptor, the virus or one or more of the related group of viruses, and the immobilized primary receptor;

(c) separating the final insoluble complex from the fluid sample and any unreacted labelled soluble receptor;

(d) measuring either the amount of label associated with the immobilized primary receptor or the amount of unreacted label; and

(e) determining the presence of the virus or the related group of viruses, in the fluid sample by relating the measurement of (d) to a measurement of a reference sample free of the virus or the related group of viruses.

This invention further relates to a process for the determination of the presence of a virus or a related group of viruses, in a fluid sample, which comprises the steps of:

(a) incubating the fluid sample with (i) an immobilized primary receptor for the virus or the related group of viruses, the immobilized primary receptor being insoluble in the fluid sample; and (ii) a labelled soluble primary receptor for the virus or the related group of viruses, to form a final insoluble complex of the labelled soluble receptor, the virus or one or more of the related group of viruses, and the immobilized primary receptor;

(b) separating the final insoluble complex from the fluid sample and any unreacted labelled soluble primary receptor;

(c) measuring either the amount of label associated with the immobilized primary receptor or the amount of unreacted label; and

(d) determining the presence of the virus or the related group of viruses, in the fluid sample by relating the measurement of (c) to a measurement of a reference sample free of the virus or the related group of viruses.

Brief Description of the Drawings

Figure 1 shows the ability of biotinylated sICAM-1 to detect a major rhinovirus 54 using several dose concentrations of biotinylated sICAM-1 with overnight incubation of virus at 30,000 $TCID_{50}$ [●] and overnight incubation at 10,000 $TCID_{50}$ [▲]. Results are expressed as the Mean ± SD of three (3) replicate cultures.

Figure 2 shows the effect of virus incubation time on the ability of sICAM-1 (100 $\mu$g/ml) to detect Rhinovirus 54 in ELISA. A 6,000 $TCID_{50}$ titer of rhinovirus 54 was used for all incubation times shown. Results are expressed as the Mean ± SD of three (3) replicate cultures.

Figure 3 shows the effect of virus concentration on the detection of rhinovirus 54 [▲] and rhinovirus 2 [●]. Biotinylated sICAM-1 was at 100 $\mu$g/ml. Results are expressed as the Mean ± SD of three (3) replicate cultures.

Figure 4 shows the use of an ABC peroxidase kit for the enhancement of detection of rhinovirus 34. sICAM-1 is used at 100 $\mu$g/ml. SA-HRP alone (0); ABC peroxidase kit utilizing biotinylated horseradish peroxidase (●). Results are expressed as the Mean ± SD of three (3) replicate cultures.

Detailed Description of the Invention

The term "primary receptor" as used herein, means the specific cell surface recognition structure which facilitates initial cell adherence of a virus or a related group of viruses.

The terms "major group rhinoviruses" and "major subgroup of rhinoviruses" as used herein, mean the human rhinoviruses which use intercellular adhesion molecule-1 (ICAM-1) as a primary receptor. For a general discussion of the ICAM-1 molecule, see Rothlein et al, J. Immunol. 137: 1270 (1986), herein incorporated by reference.

The terms "minor group rhinoviruses" and "minor subgroup of rhinoviruses" as used herein, mean the human rhinoviruses which use a primary receptor other than ICAM-1.

The term "related group of viruses" as used herein, means any group of viruses with at least one common primary receptor.

The immobilized primary receptor is preferably immobilized on a solid support and is preferably in soluble form prior to immobilization onto the solid support. The labelled soluble primary receptor is preferably in soluble form prior to being labelled. Soluble primary receptor can be prepared by chemical or enzymatic cleavage of the receptor from the cell surface or by truncation via recombinant DNA methodology. See, e.g., Marlin et al.

The solid support can be any of the known support materials useful in prior art assays, such as cellulose, sepharose, polystyrene, nylon, polyacrylamide, latex, glass, magnetizable particles, nitrocellulose, etc. Preferably, the solid support is polystyrene. A primary receptor can be immobilized onto the solid support by any procedure which produces an immobilized primary receptor capable of being bound by the target virus. For example, the primary receptor can be adsorbed in microtiter wells as described in Example 1 below and in Erlich et al, Methods in Enzymology 68: 443 (1979), or can be immobilized on a solid support using a bifunctional reagent as described in Kagedal et al, Clinica Chimica Acta 78: 103 (1977).

The amount of immobilized primary receptor utilized in the method of this invention must be sufficient to bind a detectable quantity of the target virus. This amount will vary depending upon the target virus, primary receptor, label used, etc., and should be determined empirically. In general, it is preferred that about

10μg/ml to about 20μg/ml of primary receptor immobilized on the solid support per 50μl of fluid sample, be utilized.

A primary receptor can be labeled by known means (e.g., with enzymatic, fluorogenic, radiometric, bioluminescent, chemiluminescent, colorimetric, etc., labels and markers), provided that the label does not have a deleterious effect on the binding of the primary receptor to the target virus. For example, the procedure described in Example 1 can be used in biotinylating a primary receptor for the purposes of this invention, or a procedure such as the one described in Woodhead et al, Clinical Chemistry 29(8): 1474 (1983), can be used in labeling a primary receptor for the purposes of this invention. The amount of labelled soluble receptor utilized in the method of this invention must be sufficient to permit the detection of the virus bound by the labelled soluble receptor. This amount will vary depending primarily upon the type of label used and should be determined empirically. Preferably, the primary receptor is biotinylated using a procedure as described in Example 1. The bound biotinylated primary receptor is then detected by using a streptavidin enzyme conjugate, such as a streptavidin peroxidase conjugate.

The final insoluble complex of labelled soluble receptor, virus and immobilized receptor bound to a solid support, can be produced using three assay procedures, referred to as a forward assay, a reverse assay, and a simultaneous assay. In the forward assay, the fluid sample is first incubated with an immobilized receptor for an appropriate period of time to form a first insoluble complex and then the first insoluble complex so formed is incubated with a labelled soluble receptor for an appropriate period of time to form the final insoluble complex. In the forward assay, the immobilized receptor and the labelled soluble receptor can be the same primary receptor or two different primary receptors for the same target virus or related group of viruses, which do not interfere with the binding of each other to the target virus or related group of viruses.

In the reverse assay, the fluid sample is first incubated with a labelled soluble receptor for an appropriate period of time to form a soluble complex of labelled soluble receptor and virus. The soluble complex so formed is then incubated with an immobilized receptor for an appropriate period of time to form the final insoluble complex. In the reverse assay, the immobilized receptor and the labelled soluble receptor should be two primary receptors for the same target virus or related group of viruses, which do not interfere with the binding of each other to the target virus or related group of viruses.

In the simultaneous assay, the fluid sample is incubated with a labelled soluble receptor and an immobilized receptor at the same time to form the final insoluble complex. In the simultaneous assay, the immobilized receptor and the labelled soluble receptor should be two primary receptors for the same target virus or related group of viruses, which do not interfere with the binding of each other to the target virus or related group of viruses.

The incubation conditions for each of the steps of the forward, reverse and simultaneous assays can vary, depending on time, temperature, and final incubation volume, but, preferably, each incubation step should be conducted for at least 15 minutes, preferably for 30 minutes or longer, at room temperature.

After the incubation, the final insoluble complex is separated from the incubation medium (i.e., fluid sample, unbound labelled soluble receptor, etc.). Since the final insoluble complex is insoluble in the incubation medium, it can be separated from the incubation medium by conventional means.

The uptake of labelled soluble receptor is directly related to the presence of the target virus bound to the complex. The amount of label associated with the final insoluble complex can be determined by at least two methods: (1) direct quantitation of the label associated with the final insoluble complex, or (2) indirect quantitation of the label remaining in the incubation medium after separation and then subtracting the amount from the total label offered. The appropriate quantitation procedure will depend largely on the label used.

The amount of label determined to be associated with the final insoluble complex in the method of this invention is then compared to the amount of label determined to be associated with the final insoluble complex of a reference sample obtained using the same label, procedure and reaction conditions that are used to obtain the signal from the fluid sample. The reference sample is free of the target virus or of any member of the target group of related viruses, and any label detected in the reference sample is considered background signal. The detection of signal in the fluid sample over the background signal of the reference sample indicates the presence of the target virus or of a member of the target group of related viruses in the fluid sample.

In a preferred embodiment of the method of this invention, the target group of related viruses are major group rhinoviruses, the immobilized primary receptor is sICAM-1 immobilized on polystyrene, the labelled soluble primary receptor is biotinylated sICAM-1, and the assay utilized is the forward assay.

The following example illustrates the method of this invention for the detection of the major group rhinoviruses.

## EXAMPLE 1

### Detection of Major Group Rhinoviruses Using sICAM

#### A. Cell Lines

HeLa cells (human carcinoma of the cervix; CCL2 F 6333), were obtained from The American Type Culture Collection (ATCC), Rockville, MD. The cells were maintained in RPMI-1640 culture medium supplemented with 50 $\mu$g/ml gentamycin, 1 mM L-glutamine (GIBCO, Grand Island, NY) and 10% heat-inactivated (56°C, 30 minutes) fetal calf serum (FCS) specifically screened for mycoplasma and viruses (Whittaker, M.A. Bioproducts, Walkersville, MD). The cells were seeded into 75cm$^2$ tissue culture flasks (Corning) and were removed by incubating the monolayers when 90% confluent with 0.25% trypsin and 0.1mM EDTA (GIBCO) at 37°C for 5 minutes. After trypsinization, the cells were washed 1X by centrifugation (500 x g) and plated into 96 well flat-bottom microtiter plates (Linbro No. 76-003-05) at 20,500 cells per culture well. Twenty-four hours later the cells reached 80-90% confluence and were challenged with the pretitered viruses described below.

#### B. Virus Stocks

The viruses used are listed in Table 1 below.

Table 1

| Summary of Virus Stocks | | | |
|---|---|---|---|
| Virus | Family | Source | HRV-Receptor[a] |
| Coxsackie A13 | Picornavirus | ATCC VR-1019 | Major |
| Rhinovirus 1B | Picornavirus | ATCC VR-481 | Minor |
| Rhinovirus 2 | Picornavirus | ATCC VR-482 | Minor |
| Rhinovirus 34 | Picornavirus | ATCC VR-1144 | Major |
| Rhinovirus 49 | Picornavirus | ATCC VR-516 | Minor |
| Rhinovirus 54 | Picornavirus | ATCC VR-521 | Major |
| Rhinovirus 89 | Picornavirus | ATCC VR-1199 | Major |

[a]Major: ICAM-1
Minor: non-ICAM-1 receptor

The virus stocks were amplified and expanded in the HeLa cells prepared in A, by infecting HeLa cells with the virus in RPMI-1640 media supplemented with 5% FCS and then freezing the infected cells at -80°C. For rhinoviruses, the medium was further supplemented with 20mM $MgCl_2$.

All the virus stocks except Coxsackie A13, were concentrated using a modification of the method described in Abraham et al, J. of Virol. 54: 409 (1984). Briefly, HeLa cells were infected with a particular rhinovirus for 4-6 hours in RPMI-1640 media containing 1% fetal bovine serum followed by incubation in media containing 2% fetal bovine serum. The culture media so produced was then frozen at -80°C after a generalized cytopathic effect was observed (18-48 hours) and thawed at 33°C. The virus supernatant was then precipitated with polyethylene glycol and pelleted through a 30% sucrose cushion (34,900 rpm for 2 hours in a Beckman SW41 rotor). The bottom 0.5ml was then vortexed, pooled, and stored in aliquots at -80°C.

C. Cytopathogenic Effect (CPE) Assay

For all viruses, HeLa cells were plated at 20,500 cells per culture well. Twenty-four (24) hours later the medium was removed and replaced with culture medium (RPMI-1640 supplemented with 5% FCS; rhinovirus medium was further supplemented with 20mM $MgCl_2$) containing dilutions of the expanded HeLa-virus stocks prepared in B. The plates were incubated at 37°C (Coxsackie) or 33°C (rhinoviruses) in 5% $CO_2$-humidified air and were stationery for 96 hours. CPE becomes apparent at 24- 72 hours by inspection using an inverted microscope. CPE was scored visually at these points in a qualitative manner. The quantitative read-out was analyzed at 96 hours after direct virus challenge in the following manner: the medium was aspirated off and 45 $\mu$l of 0.5% crystal violet in 20% methanol was added to each culture well for 5 minutes. The plates were then vigorously washed with water, air dried, and read on microplate reader (Dynatech MR600) at 570 nM. A standard curve plot of cell number vs. optical density (O.D.) was used to determine the number of viable cells left in each culture well. Percent reduction of CPE was determined as follows:

$$\frac{\text{O.D. (Antibody + Virus)} - \text{O.D. (Virus alone)}}{\text{O.D. (Medium alone)} - \text{O.D. (Virus alone)}} \times 100$$

The titers of virus stocks were analyzed at 96 hours by determining the dose of virus that causes a 50% CPE as determined by linear regression. Unless otherwise specified, 100 times the tissue culture infectious dose that causes a 50% CPE ($100TCID_{50}$) was used.

D. CPE Assay Using Biotinylated sICAM-1

sICAM-1 was prepared as described in Marlin et al.
sICAM-1 was biotinylated in the following manner: 4200$\mu$g of sICAM-1 was dissolved in 2.8ml of 100mM sodium acetate buffer, pH 5.5, and dialysed at 4°C overnight. The dialysed sICAM-1 solution was recovered and measured, and then incubated with a 5mM final sodium periodate solution for 30 minutes at 0°C. The resultant sICAM-1/periodate solution was dialysed overnight at 4°C. The dialysed sICAM-1 solution was recovered and measured, and then incubated with 1mM final biotin-LC-hydrazine (Pierce #21340) for 5 hours at room temperature. The biotinylated sICAM-1 so produced was dialysed overnight vs PBS at 4°C. The dialysed biotinylated sICAM-1 was recovered, measured, aliquoted and then stored at -80°C.

Biotinylated sICAM-1 was tested in a viral CPE assay using the procedure described in C above, using various minor and major rhinoviruses at 100 $TCID_{50}$, with and without 25$\mu$g/ml of sICAM-1.

The results of this testing are listed in Table 2 below.

Table 2

| Effect of Biotinylated sICAM-1 on Inhibition of Picornavirus Cytopathogenicity | | |
|---|---|---|
| Virus Strain | Receptor Group | % Inhibition CPE[a] |
| Rhinovirus 54 | Major | 92 |
| Rhinovirus 34 | Major | 100 |
| Rhinovirus 89 | Major | 56 |
| Coxsackie A13 | Major | 100 |
| Rhinovirus 2 | Minor | 0 |
| Rhinovirus 1B | Minor | 0 |
| Rhinovirus 49 | Minor | 0 |

[a]CPE determined on Hela cell monolayers with 100 $TCID_{50}$ of all virus strains; biotinylated sICAM-1 at 50$\mu$g/ml.

These results demonstrate that the biotinylated sICAM-1 was active in inhibiting major group rhinoviruses and was not active against minor group viruses.

E. Rhinovirus Detection Using a Forward Assay

sICAM-1 was immobilized in the following manner: 50$\mu$l of sICAM-1 solution (20$\mu$g/ml dissolved in PBS) was pipetted into the bottom of each of NUNC maxisorp 96-wells and incubated at 37°C for 60 minutes. Each well was then washed with 200$\mu$l of phosphate buffered saline (PBS), pH7.5.

i) A forward assay was conducted in the following manner: 200$\mu$l of 2% boving serum albumin/phosphate buffered saline (BSA-PBS) was added to each of the NUNC maxisorp 96-wells containing the immobilized sICAM-1 and incubated at 37°C for 60 minutes. This constitutes the blocking step. Virus, as prepared in B above, was diluted in appropriate diluents, added to the wells, and incubated at 4°C or at room temperature (RT) for 2-5 hours. The wells were then washed 5X with 200$\mu$l PBS. 50$\mu$l of the biotinylated sICAM-1 prepared above, was then added to each well and incubated at room temperature for 30 minutes. 50$\mu$l of streptavidan peroxidase conjugate (SA-HRP) (1:4000 suggested working stock) (Zymed #43-4323) was added to each well and incubated at room temperature for 30 minutes. The wells were then washed in 200$\mu$l of PBS. 200$\mu$l of the substrate, 2,2-azinodi(3-ethylbenzthiazoline) sulfonic acid (ABTS) (manufacturer's solution) (Zymed #00-2011), was then added to each well and incubated at room temperature for 10-240 minutes. The optical density of each of the wells was then determined at 410nM on a Dynatech MR600 plate reader.

The results of this assay are listed in Table 3 below.

Table 3

| Detection of Major But Not Minor Rhinoviruses With Biotinylated sICAM-1 | | |
|---|---|---|
| Virus Strain | Receptor Group | Optical Density[a] |
| | | Mean ± SD |
| Rhinovirus 54 | Major | 0.586 ± 0.043 |
| Rhinovirus 34 | Major | 1.014 ± 0.132 |
| Rhinovirus 89 | Major | 0.120 ± 0.014 |
| Rhinovirus 1B | Minor | <0.050 |
| Rhinovirus 2 | Minor | <0.050 |
| Rhinovirus 49 | Minor | <0.050 |

[a]Viruses were incubated overnight at 10,000 $TCID_{50}$ and detected with biotinylated sICAM-1 at 100 $\mu$g/ml as described above. Optical Density expressed as the Mean ± SD three (3) replicate cultures.

The data shows that three (3) major group rhinoviruses (34, 54 and 89) were detected while three (3) minor group rhinoviruses (1B, 2 and 49) did not show any significant color development.

ii) A variation of the above-described forward assay procedure was used wherein an ABC peroxidase kit (Pierce #32052) was used instead of the SA-HRP. The kit was used according to manufacture instructions except that the dilutions were made in 1% BSA-PBS.

The results of this assay are listed in Table 4 below.

Table 4

| Enhancement of Detection of Rhinovirus 34 With the ABC Peroxidase Kit | | |
|---|---|---|
| Virus Incubation Time[a] | Optical Density[b] SA-HRP | Optical Density[b] SA-Biotin-HRP |
| 1.5 Hours | 0.110 | 0.372 |
| 3.0 Hours | 0.243 | 0.762 |
| 6.0 Hours | 0.431 | 1.420 |
| 24.0 Hours | 0.504 | 1.692 |

[a]Rhinovirus 34 at 100 $TCID_{50}$ and biotinylated sICAM-1 at 75 $\mu$g/ml

[b]SA-HRP = Strepavidin horseradish peroxidase; SA-Biotin-HRP = Strepavidin biotinylated horseradish peroxidase (ABC kit).

The ABC peroxidase reagent (SA-Biotin-HRP) amplified the signal at all virus trapping incubation times and enhanced the response at the shortest incubation times, allowing for a higher signal sooner.

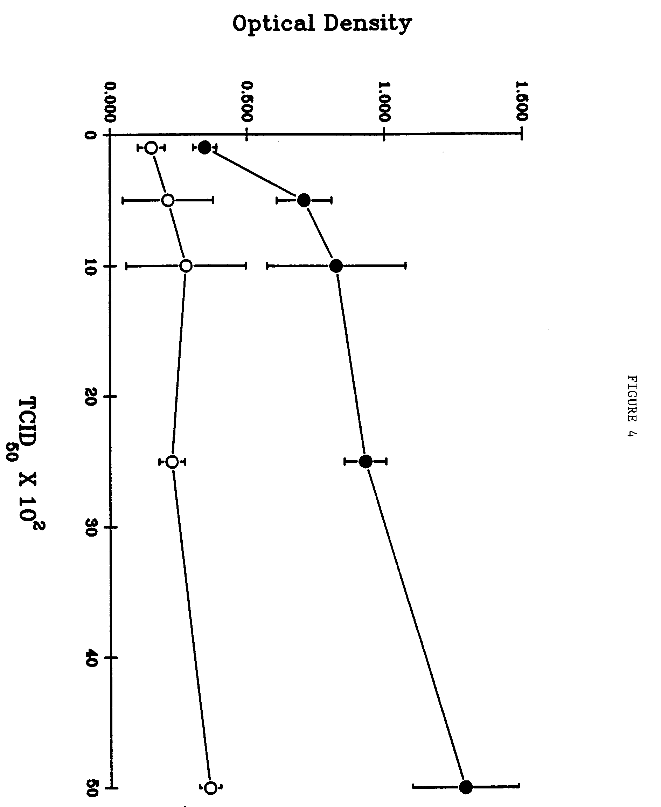

iii) An assay was performed with a 3 hour virus (rhinovirus 34) incubation period but with varied virus concentrations, using the procedure described in E(i) and E(ii) above. The results of this assay are shown in Figure 4.

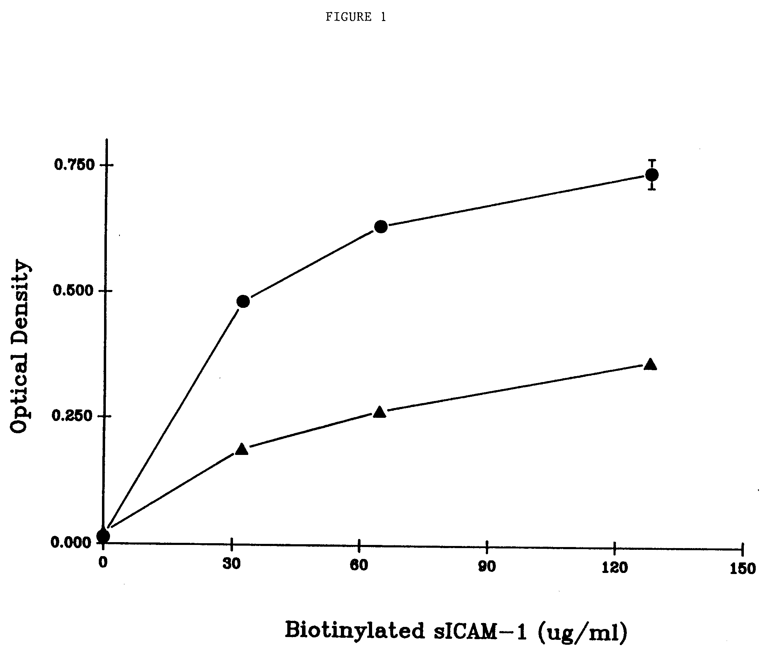

iv) HRV54, at two different concentrations, was trapped after an overnight incubation by immobilized sICAM-1 and detected by the addition of biotinylated sICAM-1, SA-HRP and substrate using the procedure described in E(i) above. The results of this assay are shown in Figure 1. The data showed that the results were dependent upon virus concentration.

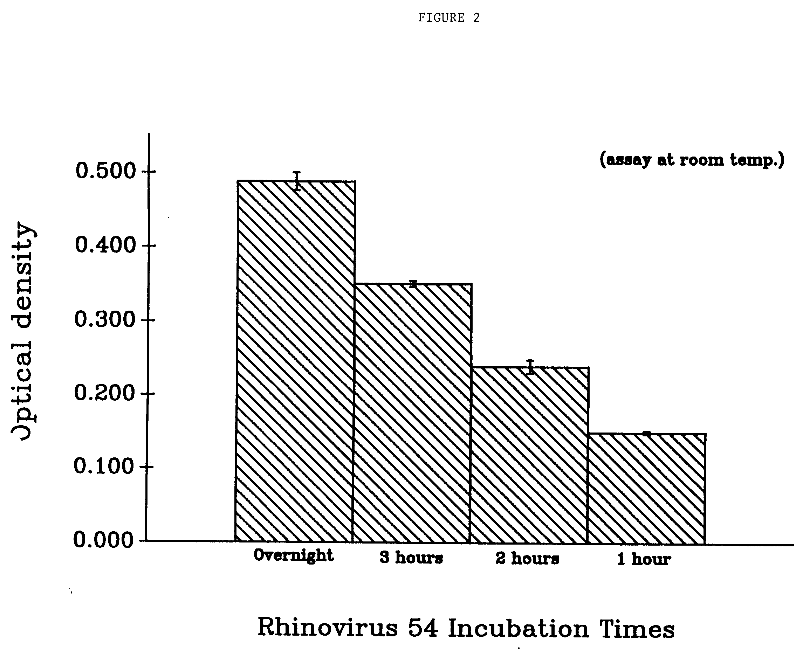

v) Rhinovirus 54 was detected at times as short as 1 hour after being trapped by sICAM-1 using the procedure described in E(i) above. The results of this assay are shown in Figure 2.

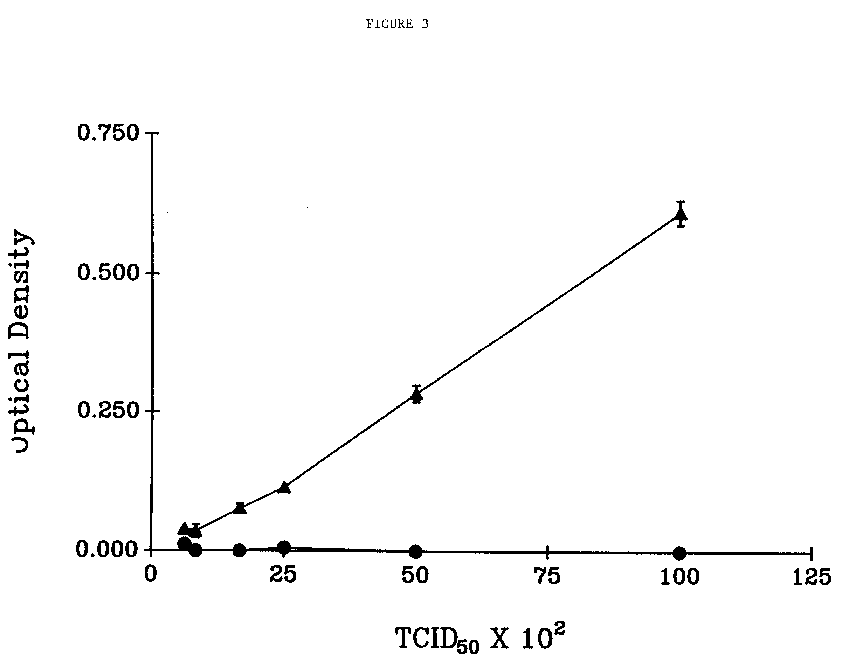

vi) Rhinovirus 54 was detected at a concentration as low as 1000 $TCID_{50}$ and rhinovirus 2 was not detected at any tested concentration. Both rhinovirus 54 and rhinovirus 2 were trapped after a 3 hour incubation with sICAM-1 using the procedure described in E(i) above. The results of this assay are shown in Figure 3.

## Claims

1. A method for the determination of the presence of a virus or a related group of viruses, in a fluid sample, which comprises the steps of:

(a) incubating the fluid sample with an immobilized primary receptor for the virus or the related group of viruses, the immobilized primary receptor being insoluble in the fluid sample, to form a first insoluble complex of the virus or one or more of the related group of viruses, and the immobilized primary receptor;

(b) incubating the first insoluble complex with a labelled soluble primary receptor for the virus or the related group of viruses, to form a final insoluble complex of the labelled soluble receptor, the virus or one or more of the related group of viruses, and the immobilized primary receptor;

(c) separating the final insoluble complex from the fluid sample and any unreacted labelled soluble receptor;

(d) measuring either the amount of label associated with the immobilized primary receptor or the amount of unreacted label; and

(e) determining the presence of the virus or the related group of viruses, in the fluid sample, by relating the measurement of (d) to a measurement of a reference sample free of the virus or the related group of viruses.

2. A method for the determination of the presence of a virus or a related group of viruses, in a fluid sample, which comprises the steps of:

(a) incubating the fluid sample with a labelled soluble primary receptor for the virus or the related group of viruses, to form a soluble complex of the virus or one or more of the related group of viruses, and labelled soluble receptor;

(b) incubating the soluble complex of (a) with an immobilized primary receptor for the virus or the related group of viruses, the immobilized primary receptor being insoluble in the fluid sample, to form a final insoluble complex of the labelled soluble receptor, the virus or one or more of the related viruses, and the immobilized primary receptor;

(c) separating the final insoluble complex from the fluid sample and any unreacted labelled soluble receptor;

(d) measuring either the amount of label associated with the immobilized primary receptor or the amount of unreacted label; and

(e) determining the presence of the virus or the related group of viruses, in the fluid sample, by relating the measurement of (d) to a measurement of a reference sample free of the virus or the related group of viruses.

3. A method for the determination of the presence of a virus or a related group of viruses, in a fluid sample, which comprises the steps of:

(a) incubating the fluid sample with (i) an immobilized primary receptor for the virus or the related group of viruses, the immobilized primary receptor being insoluble in the fluid sample; and (ii) a labelled soluble primary receptor for the virus or the related group of viruses, to form a final insoluble complex of the labelled soluble receptor, the virus or one or more of the related group of viruses, and the immobilized primary receptor;

(b) separating the final insoluble complex from the fluid sample and any unreacted labelled soluble receptor;

(c) measuring either the amount of label associated with the immobilized primary receptor or the amount of unreacted label; and

(d) determining the presence of the virus or the related group of viruses, in the fluid sample, by relating the measurement of (c) to a measurement of a reference sample free of the virus or the related group of viruses.

4. A method as recited in anyone of the claims 1 to 3 wherein the immobilized primary receptor is immobilized sICAM-1.

5. A method as recited in anyone of claims 1 to 3 or claim 4 when dependant on claim 1 or 2 wherein the labelled soluble primary receptor is labelled sICAM-1.

6. A method as recited in claim 5 wherein the labelled sICAM-1 is biotinylated sICAM-1.

7. A method as recited in anyone of the preceeding claims, wherein the immobilized primary receptor is immobilized on a solid support.

8. A method as recited in anyone of the preceeding claims, wherein the related group of viruses is the major subgroup of rhinoviruses.

FIGURE 1

EP 0 510 483 A1

Rhinovirus 54 Incubation Times

(assay at room temp.)

FIGURE 2

FIGURE 3

13

FIGURE 4

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| X | EP-A-0 391 088 (CENTER FOR BLOOD RESEARCH LABORATORIES, INC.) <br> * page 11, line 44 - page 12, line 53 * <br> --- | 1-5,7,8 | G01N33/569 |
| A | EP-A-0 319 815 (MOLECULAR THERAPEUTICS, INC) <br> * column 11, line 3 - column 13, line 20 * <br> --- | 1-3,7,8 | |
| A | EP-A-0 160 900 (ABBOTT LABORATORIES) <br> * page 2, line 30 - page 5, line 12 * <br> --- | 1-3,6 | |
| D,A | NATURE. <br> vol. 344, 1 March 1990, LONDON GB <br> pages 70 - 72; <br> S.D.MARLIN ET AL: 'A soluble form of intercellular adhesion molecule-1 inhibits rhinovirus infection' <br> * the whole document * <br> --- | 1,4,5,8 | |
| A | WO-A-8 903 222 (DANA-FABER CANCER INSTITUTE) <br> * page 2, line 28 - page 5, line 7 * <br> * page 49, line 13 - line 30 * <br><br> ----- | 1 | TECHNICAL FIELDS SEARCHED (Int. Cl.5 ) <br><br> G01N <br> C07K |

The present search report has been drawn up for all claims

| Place of search | Date of completion of the search | Examiner |
|---|---|---|
| BERLIN | 04 JUNE 1992 | DE KOK A.J. |

EPO FORM 1503 03.82 (P0401)